# EUROPÄISCHE PATENTSCHRIFT

(11) **EP 2 025 351 B1**
(45) Veröffentlichungstag und Bekanntmachung des Hinweises auf die Patenterteilung: **10.03.2010**
(21) Anmeldenummer: 08104989.2
(22) Anmeldetag: 07.08.2008
(51) Int. Cl.: A61L 9/014, A61L 9/015, A61L 9/16, A61L 9/22, B60H 3/00, B60H 3/06

(54) **Luftreinigungsvorrichtung mit O3-Neutralisierer und Luftreinigungsverfahren**
Air purification device with O3 neutraliser and air purification method
Dispositif de purification d'air doté d'un neutraliseur d'O3 et procédé de purification d'air

(30) Priorität: 08.08.2007 DE 102007037440
(43) Veröffentlichungstag der Anmeldung: 18.02.2009
(73) Patentinhaber: Meltem Wärmerückgewinnung GmbH & Co. KG, 82239 Alling (DE)
(72) Erfinder: Reynartz, Armin, 82239, Alling (DE)
(74) Vertreter: Moore, Joanne Camilla

(56) Entgegenhaltungen:
- WO-A-01/66258
- WO-A-98/21791
- WO-A-03/080375
- CA-A1- 2 534 700
- JP-A- 2004 305 395

## Beschreibung

Die Erfindung betrifft eine Luftreinigungsvorrichtung mit 03-Neutralisierer und ein Luftreinigungsverfahren. Die Luftreinigungsvorrichtung weist ein Gebläse und einen 03-Erzeuger auf. Ein derartiger 03-Erzeuger bildet Ozonmoleküle, die ein starkes Oxidationsmittel sind.

Aufgrund dieser oxidierenden Wirkung sind Ozonmoleküle für den Menschen giftig, wenn ein Wert von 0,2 mg/m³ überschritten wird. Bereits bei einer geringen Ozonaufnahme ist dieses häufig mit heftigem Schläfenkopfschmerz verbunden. Aufgrund der oxidierenden Wirkung auf die Nasenschleimhaut riecht das Ozongas in hohen Konzentrationen stechend scharf. In geringen Konzentrationen ist es praktisch geruchlos. Die Geruchsschwelle liegt bei 40 µg/m³ (Mikrogramm pro Kubikmeter).

Eine schnelle Gewöhnung an den Geruch von Ozon führt dazu, dass es nach einiger Zeit nicht mehr wahrgenommen wird. Derartige 03-Erzeuger sind weit verbreitet. Sie werden nicht nur in Klimaanlagen eingebaut, sondern sind auch in Dunstabzugshauben von Küchen vorhanden, wenn kein direkter Abzug von Küchendämpfen gewährleistet ist. 03-Erzeuger werden auch in Luftumwälzsystemen für Bürohäuser, Kaufhäuser, Krankenhäuser und andere Gebäude, selbst für Produktionsanlagen eingesetzt.

Aufgrund der geringen Halbwertszeit von Ozon von lediglich fünf Stunden in einer Standardatmosphäre-Umgebung sind in herkömmlichen Luftreinigungsvorrichtungen Einrichtungen zum Begrenzen, zum Verringen oder zum Vernichten von Restozonkonzentrationen in der Raumluft, um den menschlichen Körper nicht zu belasten, nicht bekannt. Dieses birgt jedoch die Gefahr, dass wiederholt kurzzeitig Grenzwerte der Czonbelastung von herkömmlichen Luftreinigungsvorrichtungen überschritten werden und somit die Gesundheit der Menschen, die der Raumluft ausgesetzt sind, beeinträchtigt wird.

Relevante Dokumente des Stands der Technik stellen die Druckschriften WO98/21791 A, JP 2004 305395 A, WO 03/080375 A, CA-A1-2 534 700 und WO 01/66258 dar.

Aus der Druckschrift WO 98/19961 ist eine Vorrichtung und ein Verfahren zur Erzeugung und Verwendung von Ozon bekannt. Bei dieser Vorrichtung wird Ozon in hoher Konzentration, und damit in toxischer Konzentration für die Menschen vor Ort produziert, wobei es darum geht, begrenzte Volumina von unterschiedlichen Schadstoffen, Keimen oder Molekülen in gasförmiger, flüssiger oder fester Form in abgegrenzten Volumina zu befreien. Schutzmaßnahmen gegen ein übermäßiges Aufnehmen von Ozon durch den Menschen werden in dieser Druckschrift außer Acht gelassen, bei der es sich um die Optimierung von Erzeugungs- und Verwendungsverfahren für hochprozentige Ozongehalte handelt und eine Reduzierung des Ozongehalts nicht im Sinne des aus der Druckschrift WO 98/19961 bekannten Vorrichtung und bekannten Verfahren ist.

Aus der Druckschrift US 2003/0150222 A1 ist ein Luftbearbeitungssystem für Flugzeuge bekannt, bei dem die Raumluft aus der Kabine des Flugzeugs über einen Luftleitkanal durch mehrere 03-Erzeuger geleitet wird und einer Klimaanlage zugeführt wird, in der die Passagierkabine mit verdichteter Außenluft gemischt und wieder zurück in die Passagierkabine geführt wird. Außerdem sind in dem Luftkanal alternativ Luftionisierer angeordnet, doch wird in keiner Weise ein eventuell erzeugter Überschuss an Ozonmolekülen abgebaut.

Aus der Druckschrift US 6,373,680 B1 ist ein Verfahren und eine Vorrichtung zur Ionengeneration der Luft bekannt, die auch dazu dienen kann, den Gehalt von entstehendem Ozon bei der Koronaentladung an Elektrodenspitzen zu vermindern, indem ein negativer Druckgradient auf den Ionenstrom ausgeübt wird, um damit Czonmoleküle von dem ionisierten Lufthauptstrom fernzuhalten. Eine derartige Vorrichtung hat den Nachteil, dass hier nur wenig Ozon zur Entkeimung und Reinigung von Raumluft zur Verfügung gestellt wird, zumal die vordringliche Aufgabe dieses Verfahrens und dieser Vorrichtung ist, Ionen von gewünschter Polarität für die Raumluft zur Verfügung zu stellen.

Aufgabe der Erfindung ist es, eine Luftreinigungsvorrichtung und ein Verfahren zur Luftreinigung zu schaffen, wobei ein 03-Erzeuger eingesetzt wird und dennoch die Raumluft vor übermäßigem Ozonrestgehalt geschützt ist.

Diese Aufgabe wird mit dem Gegenstand der unabhängigen Ansprüche gelöst. Vorteilhafte Weiterbildungen der Erfindung ergeben sich aus den abhängigen Ansprüchen.

Erfindungsgemäß wird eine Luftreinigungsvorrichtung mit 03-Neutralisierer und ein Luftreinigungsverfahren geschaffen. Die Luftreinigungsvorrichtung weist ein Gebläse, einen 03-Erzeuger und den 03-Neutralisierer auf. Der 03-Erzeuger und der 03-Neutralisierer sind in einem Luftleitkanal mit einer Lufteinlassöffnung und einer Luftauslassöffnung einem zu reinigenden Luftstrom des Gebläses ausgesetzt.

Eine derartige Luftreinigüngsvorrichtung hat den Vorteil, dass sie die Vorzüge einer Ozongeneration bzw. 03-Erzeugung nutzt und somit einen leistungsfähigen Geruchsentferner bildet, der gleichzeitig auch ein leistungsfähiges Desinfektionsmedium durch das erzeugte Ozon, das einen wirkungsvollen Entkeimer darstellt, schafft. Dabei können eine Vielzahl organischer Chemikalien, Bakterien und Mikroorganismen sowie ein hoher Anteil unangenehmer Gerüche durch das Ozon entfernt werden.

Durch den 03-Neutralisierer wird erreicht, dass einerseits die Ozonkonzentration gegenüber herkömmlichen Luftreinigungsanlagen und damit der Reinigungsgrad erhöht werden kann und andererseits unverbrauchte Ozonrestmoleküle sicher neutralisiert werden, sodass die Raumluft trotz erhöhtem Reinigungsgrad den Menschen nicht nachteilig belastet.

Das Gebläse kann an der Lufteinlassöffnung oder an der Luftauslassöffnung des Luftleitkanals angeordnet sein, wobei die Anordnung des Gebläses an der Lufteinlassöffnung den Vorteil aufweist, dass Partikel, die sich an den Gebläseschaufeln abgesetzt haben sowie Aerosole, die von der Lagerung des Gebläses ausgehen können durch die anschließende Anordnung eines 03-Erzeugers mit nachfolgendem O3-Neutralisierer aus dem Luftstrom entfernt werden können, was bei einer Anordnung des Gebläses an der Luftauslassöffnung nicht mehr möglich ist. Für die Anordnung des Gebläses an der Luftauslassöffnung liegt der Vorteil darin, dass die Gebläseschaufeln nur mit gereinigter Luft in Berührung kommen und somit gereinigt bleiben und sich nicht mit einer Staub- und Fettschicht überziehen können. Dazu muss jedoch die Lagerung des Gebläses aerosoldicht abgeschlossen sein.

Wegen der hohen Reaktivität des Ozons sind nur wenige Materialien gegen Ozon beständig, so dass der Luftleitkanal Chrom/ Nickelstahlbleche aufweist oder Bleche, die mit Polytetrafluorethylen, Polyvenylfluorid oder Perfluorkautschuk beschichtet sind.

Im 03-Erzeuger selbst werden die Sauerstoffmoleküle der Luft durch stille elektrische Entladung zu sauerstoffatomen dissoziiert, wonach noch im Plasma der Entladungselektroden die Ozonsynthese und eine Ozonanreicherung stattfindet. Für die stille elektrische Entladung zur Ozonerzeugung weist der 03-Erzeuger ineinander geschobene Rohrelektroden auf, zwischen denen ein poröses Dielektrikum angeordnet ist oder anstelle der Rohrelektroden Plattenelektroden, die zur Förderung der stillen elektrischen Entladung ein Dielektrikum aufweisen können. Schließlich sind für die Oberflächenentladung in Luft zur Bildung von Ozon auch drahtumwickelte Elektroden oder Spitzenelektroden geeignet. Zur Optimierung des Wirkungsgrades kann der Elektrodenabstand, die Elektrodenausrichtung, das dielektrische Material des zwischengelagerten Dielektrikums sowie die Spitzenspannung und die Spitzenfrequenz oder Impulsfrequenz, die an die Elektroden angelegt wird, variiert werden.

Dazu weist der 03-Erzeuger mindestens ein Koronaelektrodenpaar, das an eine Spannungsimpulsversorgung angeschlossen ist, auf. Dabei sind die Spitzen des Koronaelektrodenpaars und deren Abstand an die Impulsspannung, die für die elektrische Entladung zur Verfügung steht, angepasst.

Neben der stillen elektrischen Entladung mittels Elektroden können in einem 03-Erzeuger auch Ultraviolettstrahler eingesetzt werden. Dazu werden Gasentladungslampen verwendet, die einen hohen Anteil an ultraviolettem Licht abgeben und die Ozonbildung verursachen.

Durch den 03-Erzeuger wird gewährleistet, dass ausreichend Ozon erzeugt wird, um einen leistungsfähigen Geruchsentferner zu bilden und wirkungsvoll die Reduzierung von Schimmelpilzen und Mikroorganismen zu gewährleisten, was bei Herkömmlichen Luftreinigungsvorrichtungen und Verfahren eine Schwachstelle ist. Auch wird durch den 03-Erzeuger ein leistungsfähiges Desinfektionsmedium geschaffen, um wirkungsvoll den Luftstrom zu entkeimen. Schließlich wird durch das Ozon des 03-Erzeugers eine Vielzahl von organischen Chemikalien, Bakterien und Mikroorganismen und insbesondere von Gerüchen neutralisiert und die Luft wirkungsvoll von derartigen Belastungen befreit.

Durch die erhöhte Ozonkonzentration des 03-Erzeugers wird eine Verbesserung des Reinigungsgrades der Luft erreicht, wobei jedoch die Gefahr besteht, dass am Luftleitkanalauslass eine den Menschen belastende Ozonrestkonzentration auftreten könnte. Dieses wird durch einen stromabwärts des 03-Erzeugers angeordneten 03-Neutralisierers verhindert. Ein derartiger Neutralisierer kann einen Kohlefilter aufweisen, das mit Aktivkohlegranulat oder mit einem offenporigen Körper aus Aktivkohlematerial gefüllt ist. Dabei strömt die ozonhaltige Luft durch das Aktivkohlegranulat oder durch die offenen Poren des offenporigen Körpers aus Aktivkohlematerial, wobei die Ozonmoleküle zu Sauerstoffmolekülen und Kohlendioxidmolekülen reduziert werden.

Ein derartiger Aktivkohlefilter hat neben der Reduktion der Ozonmoleküle noch den zusätzlichen Effekt, dass in ihm Chemikalien, Gase, Gerüche und Zigarettenrauch abgefangen und neutralisiert werden können. Eine Regeneration des Aktivkohlefilters ist jedoch derart aufwendig, dass eine Belastung durch Chemikalien, Gase, Gerüche und Zigarettenrauch vermieden werden sollte, um die Lebensdauer des Aktivkohlefilters in Luftreinigungsgeräten zu verlängern, indem es lediglich als O3-Neutralisierer eingesetzt wird. Andererseits ist es möglich, eine Regeneration des Kohlefilters von Zeit zu Zeit durchzuführen, um ihn von Chemikalien, Rauchpartikeln und crganischen Substanzen zu befreien, indem der Kohlefilter auf beispielsweise 65 °C aufgeheizt wird, wozu der Kohlefilter mit einer Heizeinrichtung versehen sein kann, so dass flüchtige Partikel gebildet werden, die mit dem Luftstrom während der Regenerierungsphase ausgetragen werden.

Der O3-Neutralisierer kann auch ein Katalysatormetall aufweisen oder aus Granulat bestehen, das mit dem Katalysatormetall beschichtet ist. Schließlich ist es auch möglich Poren eines offenporigen Körpers mit dem Katalysatormetall zu beschichten, um dadurch wirkungsvoll die Reduzierung des Ozons zu erreichen.

In einer Ausführungsform der Erfindung weist die Luftreinigungsvorrichtung einen O3-Sensor auf, der stromabwärts des 03-Neutralisierers an der Luftauslassöffnung angeordnet ist. Dieser O3-Sensor überwacht den Ozon-Restgehalt und kann mit einem Regler zusammenwirken, der bei Überschreiten eines vorgegebenen maximalen Ozon-Schwellwertes im Luftrom an der Luftauslassöffnung den 03-Erzeuger abschaltet und bei Unterschreiten eines vorgegebenen minimalen Ozon-Schwellwertes den 03-Erzeuger einschaltet. Andererseits ist es auch möglich, auf die Spannungsversorgung des 03-Erzeugers einzuwirken und über Spannungsänderungen und Frequenzänderungen sowie Impulsfolgeänderungen den Ozongehalt unter die bekannte Geruchsschwelle von 40 µg/m³ abzusenken und zu halten.

Es ist vorgesehen, einen Mikrofilter zwischen dem 03-Erzeuger und dem 03-Neutralisierer anzuordnen. Ein derartiger Mikrofilter entfernt bis zu 99,97 % der Partikel in der zu reinigenden Luft bis zu einer minimalen Größe von beispielsweise 0,3 µm. Dadurch wird verhindert, dass sich der Neutralisator vorzeitig mit derartigen Partikeln zusetzt. Dabei entfernt der Mikrofilter feste Partikel, Rauchpartikel, Staubmilben, Hautschuppen, Pollen, Insektenkot, Schimmelpilzsporen, Tierhaare, Chemikalien, durch Ozon abgetötete Bakterien und andere neutralisierte Mikroorganismen aus der zu reinigenden Luft.

Dazu ist der Mikrofilter stromabwärts des 03-Erzeugers und stromaufwärts des 03-Neutralisierers angeordnet. Partikelgrößen, die kleiner als 0,3 µm sind, können dann von dem 03-Neutralisierer aufgenommen werden oder durch einen dem 03-Neutralisierer in Luftstromrichtung nachgeordneten Luftionisator mit nachgeschaltetem elektrostatischem Filter abgefangen werden.

Ein derartiger Luftionisierer kann Ionisationsdrähte aufweisen, die zwischen Hochspannungselektrodenplatten angeordnet sind, wobei die Ionisationsdrähte für eine Ionisierung der Luftmoleküle sorgt und die Hochspannungselektrodenplatten die Wirkung eines elektrostatischen Filters auf die ionisierten Luftmoleküle ausüben. Neben den Luftmolekülen werden jedoch auch feste Partikel, Rauchpartikel, Staubpartikel und Staubmilben, Hautschuppen, Pollen, Insektenkot, Schimmelpilzsporen, Tier- und Haushaare, Chemikalien, Bakterien, Mikroorganismen, Viren und flüchtige organische Verbindungen ionisiert und können von den Hochspannungselektrodenplatten aufgenommen und aus der Raumluft entfernt werden.

Ein weiterer Vorteil des Luftionisierers besteht darin, dass die ionisierten Luftmoleküle sich über die Auslassöffnung des Luftleitkanals in die gereinigte Raumluft verbreiten und dort für ein verbessertes gesundes Klima sorgen. Die Luftmoleküle nehmen an den Ionisationsdrähten Elektronen auf und werden somit negativ geladen, wobei ein hoher Anteil an negativen Ionen, der in der Gebirgsluft, der Meeresluft oder in der gesunden Luft nach einem Gewitter vorhanden ist, das Wohlbefinden der Menschen, die in einer derartigen ionisierten Raumluft arbeiten verbessert. Darüber hinaus sind die Luftionen in der Lage Aerosole, feste und flüssige Stoffe in der Luft wie Staubteilchen zu binden, die dadurch schwerer und elektrisch geladen werden und somit von den oben erwähnten Hochspannungsplattenelektroden aufgenommen werden können.

Die negativen Ionen, die an den Ionisierungsdrähten durch Aufnahme von Elektronen gebildet werden, verbessern das Raumklima für Menschen, zumal bei zu wenig negativen Sauerstoffionen Menschen dazu neigen, mit Nervosität, Depressionen, Schlafstörungen, Erschöpfungen, Kreislaufbeschwerden und verminderter psychischer und physischer Belastbarkeit zu reagieren. Mit einem nachgeschalteten Luftionisierer kann somit nicht nur eine saubere und gute Raumathmosphäre erreicht werden, es können auch die Umweltbelastungen gemildert werden, die Körperenergie gesteigert werden, die Widerstandsfähigkeit des menschlichen Körpers erhöht und Krankheiten vorgebeugt werden.

Außerdem wird durch den Luftionisierer nicht nur die Konzentration an negativen Ionen in der Luft zur Förderung des Wohlbefindens des Menschen erhöht, sondern durch das Anlagern der Elektroden an Feinstäuben, Keimen und Bakterien, die nicht durch den vorgeschalteten Mikrofilter abgefangen wurden, können auch diese noch aus der Raumluft entfernt werden, da die Hochspannungsplattenelektroden wie ein elektrostatischer Filter wirken. Die Filterwirkung kann durch einen weiteren nachgeschalteten elektrostatischen Filter aus entsprechenden Hochspannungsplattenelektroden noch verstärkt werden.

Dabei ist die Anzahl der Ionen/cm³ Luft bedeutend. Bei bisherigen und herkömmlichen Luftreinigungsanlagen liegt der Anteil der Ionen/cm³ bei 50, so dass physiologische Störungen beim Menschen auftreten können. Für ein gesundes Klima sind 1000 bis 2000 Ionen/cm³ eine minimale Voraussetzung. Körperliche Abwehr des Menschen und die Widerstandsfähigkeit werden bereits verbessert, wenn der Ionenanteil zwischen 5000 und 50000 Ionen/cm³ liegt. Wird der Ionenanteil auf bis zu 100000 Ionen/cm³ gesteigert, so werden Mikrobakterien getötet, so dass Infektionskrankheiten in einer in dieser Weise gereinigten und mit Luftionen angereicherten Raumluft vermindert werden. Schließlich wird bei Tonenkonzentrationen von bis zu 500000 Ionen/cm³ die Selbsterholung des menschlichen Körpers von Krankheiten gefördert, so dass eine entsprechende Ausführungsform der Erfindung mit einem derartigen Luftionisierer im Auslassbereich des Luftleitkanals besonders für Arztpraxen, Krankenhäuser und Laboratorien geeignet ist. Dabei sollten diese Luftreinigungsvorrichtungen mit Luftionisierer für geschlossene Räume im Dauerbetrieb betrieben werden, zumal die Halbwertszeit der Luftionen begrenzt ist.

In einer weiteren Ausführungsform der Erfindung weist die Luftreinigungsvorrichtung einen Luftkühler auf, der stromaufwärts des 03-Erzeugers angeordnet sein kann, um den Luftstrom durch die Luftreinigungsvorrichtung zu temperieren. Außerdem ist es vorgesehen, für die Luftreinigungsvorrichtung eine Luftheizung bereitzustellen, so dass im Zusammenwirken von Luftkühler und Luftheizung nun die Luftreinigungsvorrichtung eine Klimaanlage aufweist.

Um schließlich die Raumluft durch Frischluft zu ergänzen ist es vorgesehen, dass die Luftreinigungsvorrichtung ein Dreiwegeventil mit zwei Eingängen und einem Ausgang aufweist. Dabei ist an dem Ausgang des Dreiwegeventils der Luftkanal angeordnet. Ein erster Eingang kann mit einer zu reinigenden Raumluft und ein zweiter Eingang kann über eine Klimaanlage für Luftkühlung und/oder Luftheizung mit einer zu reinigenden Außenluft in Verbindung stehen. Eine derartige Luftreinigungsvorrichtung hat den Vorteil, dass einerseits die Wärme in der Raumluft genutzt wird und zum anderen der Raumluft je nach Bedarf temperierte Außenluft zugeführt werden kann, die jedoch ebenfalls durch den Luftleitkanal und seine reinigenden Komponenten geleitet wird. Eine derartige Luftreinigungsvorrichtung mit Luftionisierer ist für Labore, Krankenhäuser, Kliniken, Wartezimmer und Wartehallen sowie Arztpraxen zur Bereitstellung einer Raumhygiene geeignet.

Reinigungsvorrichtungen ohne Ionisierer sind für Küchen, Großküchen, Fitnessstudios, Wohn- und Schlafräume geeignet. Luftreinigungsvorrichtungen mit einem 03-Erzeuger, der eine erhöhte Ozonkonzentration bereitstellt, wird in Entsorgungsbetrieben, bei Brandschäden, in der Asphaltindustrie, in Toilettenbereichen und bei Wasserschäden zur Beseitigung von Fäulnisgasen, Fäulnisgeruch und Schimmel eingesetzt. Schließlich können die erfindungsgemäßen Luftreinigungsanlagen auch den Erfordernissen von Tierhaltungsbetrieben, Tierverwertungsbetrieben, von Fischtheken und Fleischtheken, von Klär- und Kompostieranlagen, von Kühlräumen für Fisch, Fleisch, Käse, Butter, Eier, Obst und Gemüse angepasst werden. Auch in diesen Fällen ist eine erhöhte Ozonkonzentration durch den 03-Erzeuger notwendig, die aber auf eine ungefährliche Ozonkonzentration durch den O3-Neutralisierer reduziert werden kann.

Schließlich können mit entsprechender Reinigungskapazität erfindungsgemäße Luftreinigungsvorrichtungen in Produktionsräumen, in Büroräumen, in Räumen von chemischen Reinigungen, in Gaststätten und Frisiersalons sowie in Verkehrsmitteln eingesetzt werden.

Ein Luftreinigungsverfahren unter Verwendung einer Luftreinigungsvorrichtung mit einem Gebläse, einem 03-Erzeuger und einem 03-Neutralisierer weist nachfolgende Verfahrensschritte auf. Zunächst wird das Gebläse eingeschaltet, das einen Luftstrom durch einen Luftleitkanal von einer Lufteinlassöffnung zu einer Luftauslassöffnung erzeugt. Dabei kann das Gebläse sowohl am Lufteinlass als auch am Luftauslass angeordnet sein. Danach erfolgt ein Leiten des Luftstroms durch einen 03-Erzeuger unter Bilden von Ozonmolekülen, die organische und anorganische Partikel sowie Mikroorganismen zu geruchsneutralen Partikeln oxidieren. Nach dem Durchleiten durch den 03-Erzeuger wird die Luft durch einen 03-Neutralisierer unter Reduktion der Ozonrestmoleküle zu geruchsfreien O₂-Sauerstoffmolekühlen geleitet.

Ein derartiges Verfahren hat den Vorteil, dass in der gereinigten Raumluft die Ozonkonzentration unter einen minimalen Schwellenwert abgesenkt werden kann, so dass sich die Toxizität des Ozons nicht auswirkt. Andererseits hat dieses Verfahren den Vorteil, dass die Konzentration an Ozonmolekülen in der Luft im Luftleitkanal durch den 03-Erzeuger derart erhöht werden kann, dass eine sichere Vernichtung von organischen und anorganischen Partikeln sowie Mikroorganismen zu geruchsneutralen Partikeln bzw. zu abgetöteten Mikroorganismen erreicht werden kann.

In einer weiteren Durchführungsform des Verfahrens wird der Luftstrom nach dem Durchleiten durch den 03-Erzeuger und vor dem Einbringen in den 03-Neutralisierer im Luftleitkanal durch einen Mikrofilter geführt, wobei die oxidierten Partikel und oxidierten Mikroorganismen mit Hilfe des Mikrofilters aus dem Luftstrom herausgefiltert werden und zusätzlich der Mikrofilter durch Ozonrestmoleküle entkeimt wird.

Prinzipiell wäre auch die Anordnung des Mikrofilters stromabwärts des Neutralisierers möglich, jedoch würde dann der 03-Neutralisierer einer erhöhten Belastung durch oxidierte organische und anorganische Partikel ausgesetzt, so dass sich der 03-Neutralisierer relativ frühzeitig zusetzt. Durch das Vorschalten des Mikrofilters wird der O3-Neutralisierer lediglich durch Submikrometer große Partikel belastet, während Mikrometer große Partikel bereits von dem Mikrofilter abgefangen werden. Darüber hinaus ist es vorgesehen, für den 03-Neutralisierer Oberflächen von Katalysatormetallen zur Reduktion von Ozonrestmolekühlen zu geruchsfreien O₂-Sauerstoffmolekühlen einzusetzen. Derartige Katalysatormetalle haben den Vorteil gegenüber einer Aktivkohle, dass sie die Reduktion des Ozons zu O₂-Sauerstoffmolekühlen fördern und sich selbst nicht verändern.

Weiterhin ist es vorgesehen, dass im Luftleitkanal der Luftstrom vor dem Austritt aus der Luftaustrittsöffnung ionisiert wird und Submikrometer-Partikel und Submikrometer-Mikroorganismen aus dem Luftstrom entfernt werden. Bei einer derartigen Ionisierung der Luft in dem Luftleitkanal werden Luftmoleküle beim Vorbeistreichen an Ionisierungsdrähten durch Aufnahme von Elektronen negativ aufgeladen und können durch entsprechende elektrostatische Filter bzw. Hcchspannungselektrodenplatten herausgefiltert werden, so dass selbst Submikrometer große Partikel nicht mehr in der Raumluft nach Passieren des Ionisierers oder des Ionisierers in Zusammenwirken mit einem elektrostatischen Filter vorhanden sind.

Darüber hinaus ist es vorgesehen, dass vor dem Austritt des Luftstroms der Ozongehalt des Luftstroms gemessen wird und bei Überschreiten eines vorgegebenen maximalen Ozon-Schwellwertes im Luftrom wird der 03-Erzeuger abgeschaltet und bei Unterschreiten eines vorgegebenen minimalen Ozon-Schwellwertes wird der 03-Erzeuger wieder eingeschaltet. Mit dieser Verfahrensvariante wird gewährleistet, dass die Ozonrestwerte nicht nur festgestellt werden, sondern auch auf einem niedrigen Konzentrationsniveau gehalten werden können. Darüber hinaus ist es vorgesehen, dass das Verfahren einen temperierten Luftstrom liefert. Zu dieser Temperierung wird dem Luftleitkanal eine Klimaanlage vorgeschaltet, die über einen Luftkühler und/oder eine Luftheizung verfügt.

Die Erfindung wird nun anhand der beigefügten Figuren näher erläutert.
- Figur 1: zeigt eine Prinzipskizze einer Luftreinigungsvorrichtung nicht gemäß der Erfindung;
- Figur 2: zeigt eine Prinzipskizze einer Luftreinigungsvorrichtung einer zweiten Ausführungsform der Erfindung;
- Figur 3: zeigt eine Prinzipskizze einer Luftreinigungsvorrichtung einer dritten Ausführungsform der Erfindung;
- Figur 4: zeigt eine Prinzipskizze einer Luftreinigungsvorrichtung einer vierten Ausführungsform der Erfindung;
- Figur 5: zeigt eine Prinzipskizze einer Luftreinigungsvorrichtung einer fünften Ausführungsform der Erfindung;
- Figur 6: zeigt eine Prinzipskizze einer Luftreinigungsvorrichtung einer sechsten Ausführungsform der Erfindung;
- Figur 7: zeigt eine Prinzipskizze eines Dreiwegeventils einer weitere Ausführungsform der Erfindung.

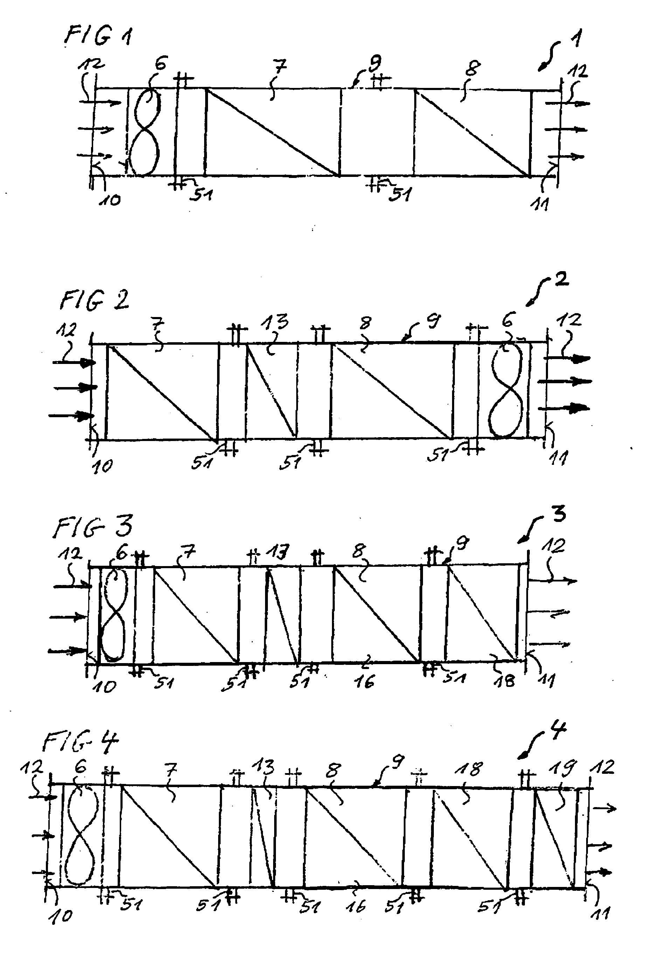

Figur 1 zeigt eine vergleichende Prinzipskizze einer Luftreinigungsvorrichtung 1 einer Ausführungsform nicht gemäß der Erfindung. Diese Luftreinigungsvorrichtung weist einen Luftleitkanal 9 auf, der eine Lufteinlassöffnung 10 und eine Luftauslassöffnung 11 besitzt. Über die Lufteinlassöffnung 10 wird in dieser Ausführungsform mit Hilfe eines Gebläses 6 ein Luftstrom 12 in den Luftleitkanal 9 geleitet, wobei der Luftstrom 12 aus einer zu reinigenden Raumluft oder Außenluft besteht. Zunächst wird der Luftstrom 12 durch einen 03-Erzeuger 7 geleitet, in dem Ozonmoleküle mit relativ hoher Konzentration gebildet werden.

Diese Ozonmoleküle oxidieren Geruchspartikel, organische und anorganische Partikel sowie Bakterien und Mikroorganismen, die sich in der Raumluft oder Außenluft befinden und wirken für lebende Organismen entkeimend. Danach wird der Luftstrom mit oxidierten Partikeln und den verbliebenen Ozonrestmolekülen durch einen O3-Neutralisierer 8 geleitet. In diesem 03-Neutralisierer 8 werden zumindest die aggressiven Ozonrestmoleküle zu geruchsfreien O₂-Sauerstoffmolekühlen reduziert. Dabei sinkt die Ozonkonzentration nach dem 03-Neutralisierer unter die Ozongeruchsschwelle von 40 µg/m³. Bei dieser ersten Ausführungsform der Erfindung wird der 03-Neutralisator mit den oxidierten Partikeln, die im 03-Erzeuger entstehen belastet.

Da die Aufnahmefähigkeit derartiger 03-Neutralisierer für Fest- und Flüssigstoffe in dem Luftstrom begrenzt ist, führt dies dazu, dass der O3-Neutralisierer bei Wartungsarbeiten auszuwechseln ist. Deshalb ist die Luftreinigungsvorrichtung modular aufgebaut, wobei die einzelnen Module Gebläse 6, 03-Erzeuger 7 und O3-Neutralisierer 8 über Flansche 51 aneinander geflanscht sind, so dass sie einzeln ausgetauscht werden können. Das Gebläse ist in dieser ersten Ausführungsform der Erfindung an der Lufteinlassöffnung 10 des Luftleitkanals angeordnet, so dass Aerosole, die beispielsweise der Gebläselagerung entweichen können in dem Luftleitkanal entfernt werden können. Andererseits besteht die Gefahr, dass sich auf den Gebläseblättern Staub- oder Fettschichten absetzen, wenn mit langen Stillstandszeiten oder hochbelasteter Luft zu rechnen ist.

Figur 2 zeigt eine Prinzipskizze einer Luftreinigungsvorrichtung 2 einer zweiten Ausführungsform der Erfindung. Komponenten mit gleichen Funktionen wie in Figur 1 werden mit gleichen Bezugszeichen gekennzeichnet und nicht extra erörtert. Der Unterschied der Luftreinigungsvorrichtung 2 gemäß Figur 2 zu der Luftreinigungsvorrichtung 1 gemäß Figur 1 besteht darin, dass zwischen dem 03-Erzeuger 7 und dem O3-Neutralisierer 8 im Luftleitkanal 9 ein Mikrofilter 13 angeordnet ist, welcher beispielsweise 99,97 % der Partikel größer als C,3 µm aus dem Luftstrom ausfiltert.

Dieser Mikrofilter 13 wird stromabwärts des 03-Erzeugers aufgebaut, so dass Mikroorganismen, die sich im Mikrofilter 13 ansammeln durch das Ozon des 03-Erzeugers noch im Mikrofilter 13 entkeimt werden können. Der Mikrofilter 13 ist wiederum durch Flansche 51 modular in die Luftreinigungsvorrichtung 2 integrierbar und ist dafür vorgesehen, dass er in unterschiedlichen Wartungsintervallen ausgewechselt oder gereinigt werden kann. Ferner ist in dieser Ausführungsform der Erfindung das Gebläse 6 an der Luftauslassöffnung 11 angeordnet, womit der Vorteil verbunden ist, dass die Gebläseschaufeln von gereinigter Luft umspült werden und somit keine Staub- oder Fettschichten ansetzten, jedoch werden dann erhöhte Anforderungen an die Aerosoldichtheit der Lagerung des Gebläserads gestellt.

Figur 3 zeigt eine Prinzipskizze einer Luftreinigungsvorrichtung 3 einer dritten Ausführungsform der Erfindung, wobei auch hier der Luftleitkanal 9 modular aufgebaut ist und zusätzlich zu den in Figur 2 gezeigten Komponenten noch stromabwärts des 03-Neutralisierers einen Luftionisierer 18 aufweist. Das Gebläse 6 ist in dieser dritten Ausführungsform der Erfindung wieder an der Lufteinlassöffnung 10 angeordnet, während gegenüberliegend vor der Luftauslassöffnung 11 der Luftionisierer 18 positioniert ist. Bei dem Luftisolierer 18 wird das Entstehen von Ozon durch entsprechend geformte und versorgte Elektroden soweit unterdrückt, dass lediglich die Luftmoleküle Elektronen von den Elektroden abnehmen oder abstreifen können und somit negativ geladen werden. Dieser mit negativ geladenen Ionen angereicherte Raumluft hat wie bereits oben ausgeführt deutliche Vorteile gegenüber den in gewerblichen oder privaten Räumen bisher üblichen Luftqualitäten.

Figur 4 zeigt eine Prinzipskizze einer Luftreinigungsvorrichtung 4 einer vierten Ausführungsform der Erfindung, wobei sich diese Ausführungsform der Erfindung von den vorhergehenden Ausführungsformen darin unterscheidet, dass zusätzlich ein elektrostatischer Luftfilter 19 vor dem Austritt des Luftstroms 12 aus dem Luftleitkanal 9 angeordnet ist. Dieser elektrostatische Luftfilter 19 weist elektrisch vorgespannte Hochspannungsplattenelektroden auf, die dafür sorgen, dass Mikro- und Submikrometer-Partikel, die nicht von dem Mikrofilter 13 aus dem Luftstrom 12 abgefangen werden, sich nun auf den Elektrodenplatten des elektrostatischen Luftfilters 19 festsetzen und in Wartungsintervallen entfernt werden können.

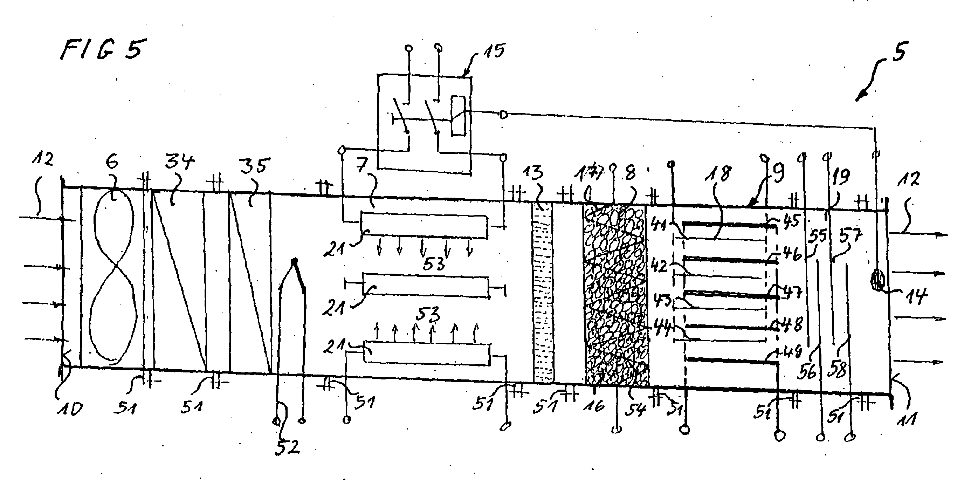

Figur 5 zeigt eine Prinzipskizze einer Luftreinigungsvorrichtung 5 einer fünften Ausführungsform der Erfindung. Komponenten mit gleichen Funktionen wie in den vorhergehenden Figuren werden mit gleichen Bezugszeichen gekennzeichnet und nicht extra erörtert. Diese Luftreinigungsvorrichtung 5 ist im Bereich ihrer Lufteinlassöffnung 10 um eine Anlage zur Lufttemperierung ergänzt. Derartige Klimaanlagen sind weit verbreitet. In dieser Ausführungsform der Erfindung weist die Klimaanlage sowohl einen Luftkühler 34 als auch eine Luftheizung 35 auf, die in Luftstromrichtung hintereinander geschaltet sind. Die Lufttemperatur wird dabei durch ein Thermoelement 52 überwacht. Das Thermoelement 52 kann mit einem nicht gezeigten Regler zusammenwirken, um den Einsatz von Luftkühler 34 und Luftheizung 35 zu regeln. Der temperierte Luftstrom 12 wird danach in einen 03-Erzeuger, der modular aufgebaut ist, geleitet, wobei der O3-Erzeuger in dieser fünften Ausführungsform der Erfindung UV-Lichtquellen 21 aufweist, die UV-Strahlen 53 erzeugen und in dem Luftstrom zur Dissoziation von O₂-Sauerstoffmolekühle und zur Bildung von Ozon-Molekülen beitragen.

Die Wirkungsweise dieser Ozon-Moleküle wurde bereits oben näher erläutert, so dass auf eine Wiederholung an dieser Stelle verzichtet wird. Die oxidierten Substanzen, werden beispielsweise zu 99,97 % anschließend durch einen offenporigen Mikrofilter 13 bis zu einer Größe von beispielsweise 0,3 Mikrometer ausgefiltert, bevor die Ozonrestmoleküle und Partikel im Submikrometerbereich mit dem Luftstrom in den stromabwärts angeordneten 03-Neutralisierer 8 gelangen. In dieser Ausführungsform der Erfindung weist der 03-Neutralisierer einen Kohlenstofffilter 16 aus Aktivkohle 54 auf, wobei die Aktivkohle 54 in dieser fünften Ausführungsform der Erfindung aus Aktivkohlegranulat besteht, durch das der Luftstrom 12 nach dem Ausfiltern von mikrometergroßen Bestandteilen geleitet wird. Das Kohlenstofffilter 16 kann nun im Luftstrom verbliebenen Submikrometerpartikel wie Chemikalien, Gase, Gerüche, Aerosole, Rauchpartikel, Pollen, Schimmelpilzsporen, abgetötete Bakterien, Viren und andere Submikrometerorganismen aufnehmen und neutralisieren. Außerdem reagiert die Aktivkohle mit den Ozonrestmolekülen und reduziert diese zu geruchfreien O₂-Sauerstoffmolekühlen.

Das Aktivkohlefilter 16 kann in entsprechenden Wartungsabständen regeneriert werden, dazu weist das Aktivkohlefilter 16 einen Heizeinrichtung 17 in Form eines Heizdrahtes oder einer Heizwicklung auf, mit der dass Aktivkohlefilter auf Regenerationstemperatur erwärmt werden kann. Die Heizwicklung kann dazu direkt im Bereich des Kohlegranulats des Aktivkchlefilter 16 angeordnet sein. Außerdem ist eine automatische Regelung für den Regenerationsvorgang vorgesehen, der automatisch dann einsetzt, wenn ein überhöhter 03- Gehalt am Filterausgang ermittelt wird.

Ferner ist stromabwärts des 03-Neutralisierers 8 in dieser Ausführungsform der Erfindung ein Luftionisierer 18 angeordnet, der zwischen Hochspannungsplattenelektroden 45 bis 49 Ionisierungsdrähte 41 bis 44 aufweist. Mit den Ionisierungsdrähten kommen Luftmoleküle in Berührung und nehmen Elektronen aus den Ionisierungsdrähten 41 bis 44 auf, so dass sie negativ geladen werden. Mit den negativ geladenen Luftmolekülen werden auch die Feinpartikel, die noch im Luftstrom verblieben sind, negativ aufgeladen und von den Hochspannungsplattenelektroden 45 bis 49 aufgenommen.

Weiterhin besitzt diese fünfte Ausführungsform der Erfindung stromabwärts des Luftionisierers 18 einen elektrostatischen Luftfilter 19 aus einander gegenüberstehenden Plattenelektroden 55 bis 57, zwischen denen der Luftstrom 12 durchgeführt wird, wobei elektrisch geladene Restpartikel von den Oberflächen der Plattenelektroden 55 bis 57 aufgenommen werden, so dass eine nahezu Partikelfreie Luft aus der Luftauslassöffnung 11 ausströmen kann. An der Luftauslassöffnung 11 ist schließlich ein Ozonsensor 14 angeordnet, der mit einem Regler 15 zusammenwirkt und bei einem minimalen Ozonwert der 0-zonkonzentration die UV-Lampen des 03-Erzeugers einschaltet und bei Überschreiten eines maximalen Schwellenwertes an Ozonmolekülen die UV-Lichtquellen 21 des 03-Erzeugers abschalten. Somit kann gewährleistet werden, dass je nach Einstellung des maximalen Schwellenwertes mindestens eine geruchsfreie Ozonkonzentration am Luftauslass 11 des Luftleitkanals 9 in den luftgereinigten Raum zurück geleitet wird.

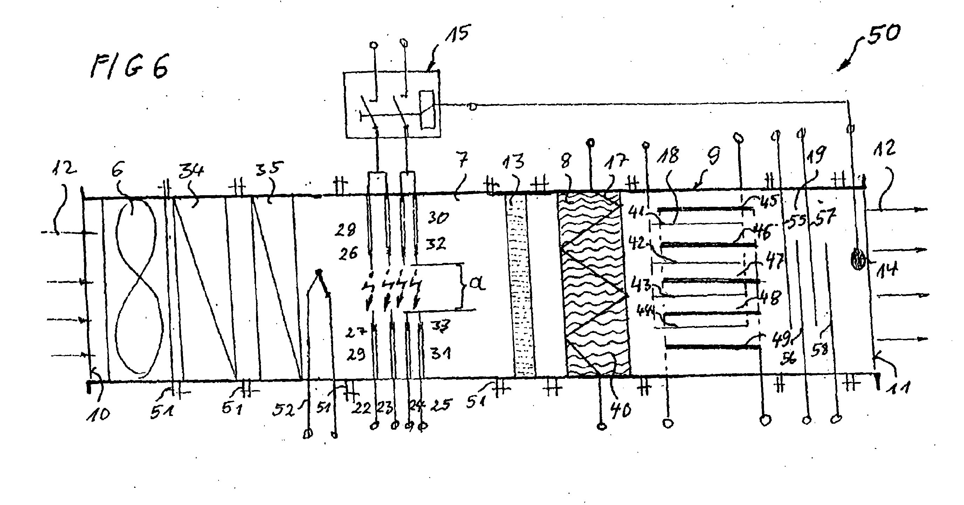

Figur 6 zeigt eine Prinzipskizze einer Luftreinigungsvorrichtung 50 gemäß einer sechsten Ausführungsform der Erfindung. Komponenten mit gleichen Funktionen wie den vorhergehenden Figuren werden mit gleichen Bezugszeichen gekennzeichnet und nicht extra erörtert. Diese sechste Ausführungsform der Erfindung unterscheidet sich von der fünften Ausführungsform der Erfindung gemäß Figur 5 dadurch, dass in dem 03-Erzeuger Koronaspitzen 26 bis 33 in einem Abstand a voneinander angeordnet sind, so dass beim Anlegen einer Spannung eine stille elektrische Entladung stattfindet, bei der an den Koronaelektrodenpaaren 22 bis 25 in dem Luftstrom 12 Ozon gebildet wird, das die oben bereits beschriebenen Wirkungen entfaltet. Ferner ist in dem 03-Neutralisierer 8 ein Katalysatormetall 40 vorgesehen, das die Reduktion von Ozonrestmolekühlen zu O₂-Sauerstoffmolekühlen bewirkt, ohne selbst verbraucht zu werden.

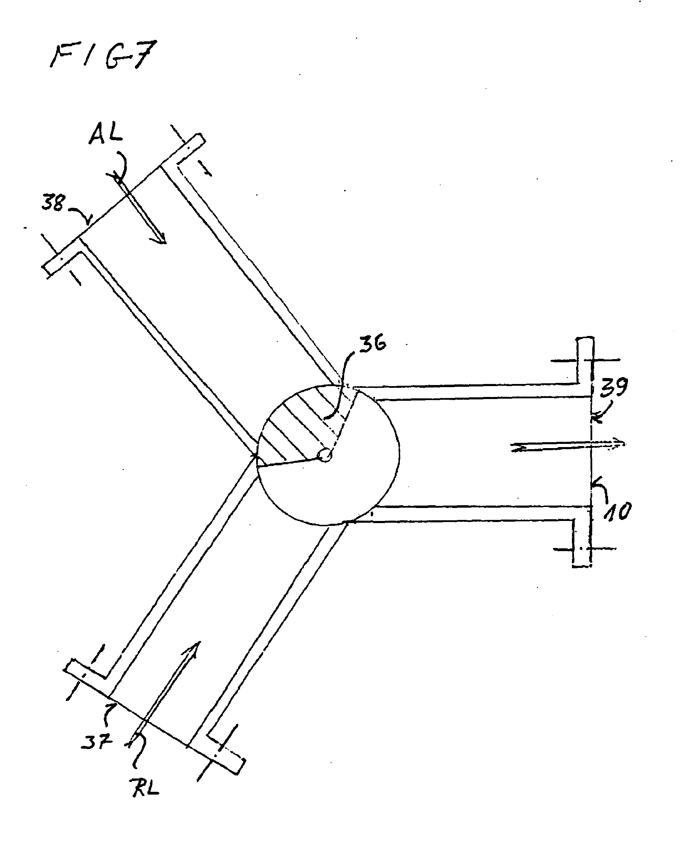

Figur 7 zeigt eine Prinzipskizze eines Dreiwegeventils 36 einer weitere Ausführungsform der Erfindung. Dieses Dreiwegeventil 36 weist zwei Eingänge 37 und 38 und einen Ausgang 39 auf. An den ersten Eingang 37 wird eine zu reinigende Raumluft RL angeschlossen, während den zweiten Eingang 38 temperierte Außenluft AL, die beispielsweise durch eine Klimaanlage temperiert ist, eingeleitet werden kann. Das Dreiwegeventil 36 kann nun, derart eingestellt werden, dass ausschließlich Raumluft RL dem Ausgang 39 zugeführt wird. In einer weiteren Einstellung kann ausschließlich Außenluft AL der Luftreinigungsvorrichtung zugeführt werden.

Ferner ist es möglich, dass eine Mischung aus Raumluft RL und temperierter Außenluft AL gebildet wird, die zum Ausgang 39 geleitet wird. An dem Ausgang 39 ist dazu die Lufteinlassöffnung 10 einer der in den vorhergehenden Ausführungsformen erörterten Luftreinigungsvorrichtungen 1 bis 5 oder 50 angeordnet, wobei die in den Ausführungsformen 5 und 6 gezeigten Luftkühler 34 bzw. Luftheizer 35 nun an den Eingang 38 des Dreiwegeventils 36 angeflanscht sein können, um temperierte Außenluft AL dem Luftreiniger zuzuführen. Wird eine derartige Vorrichtung mit einem Dreiwegeventil, einer Temperierung und einer Reinigung der Luft für Luftfahrzeugkabinen eingesetzt, so ist zusätzlich am zweiten Eingang 38 ein Luftkompressor vorgesehen, der dafür sorgt, dass die temperierte Außenluft zunächst auf den Druck der Passagierkabine gebracht wird.

Weitere Ausführungsbeispiele der Erfindung sind unter folgenden Ziffern aufgelistet:
2 Luftreinigungsvorrichtung, bei der der 03-Erzeuger stromaufwärts von dem 03-Neutralisierer im Luftleitkanal angeordnet ist.
3 Luftreinigungsvorrichtung nach Ziffer 2, bei der das Gebläse an der Lufteinlassöffnung oder an der Luftauslassöffnung angeordnet ist.
4 Luftreinigungsvorrichtung nach Ziffer 2, bei der ein Mikrofilter zwischen dem 03-Erzeuger und dem 03-Neutralisierer angeordnet ist.
5 Luftreinigungsvorrichtung nach einer der vorhergehenden Ziffern, die einen O3-Sensor aufweist, der stromabwärts des 03-Neutralisierers an der Luftauslassöffnung angeordnet ist.
6 Luftreinigungsvorrichtung nach Ziffer 5, deren O3-Sensor mit einem Regler zusammenwirkt, der bei Überschreiten eines vorgegebenen maximalen Ozon-Schwellwertes im Luftrom an der Luftauslassöffnung den 03-Erzeuger abschaltet und bei Unterschreiten eines vorgegebenen minimalen Ozon-Schwellwertes den 03-Erzeuger einschaltet.
7 Luftreinigungsvorrichtung nach einer der vorhergehenden Ziffern, deren 03-Neutralisierer einen Kohlefilter aufweist
8 Luftreinigungsvorrichtung nach Ziffer 7, deren Kohlefilter ein Aktivkohlegranulat aufweist.
9 Luftreinigungsvorrichtung nach Ziffer 7, deren Kohlefilter einen offenporigen Körper mit Aktivkohlematerial aufweist.
10 Luftreinigungsvorrichtung nach Ziffer 7, deren Kohlefilter mit einer Heizeinrichtung zur Regeneration gekoppelt ist.
11 Luftreinigungsvorrichtung nach Ziffer 2 bis 6, deren 03-Neutralisierer ein Katalysatormetall aufweist.
12 Luftreinigungsvorrichtung nach Ziffer 11, deren 03-Neutralisierer ein Granulat aufweist, dass mit dem Katalysatormetall beschichtet ist.
13 Luftreinigungsvorrichtung nach Ziffer 11, deren 03-Neutralisierer einen offenporigen Körper aufweist, dessen Poren mit dem Katalysatormetall beschichtet sind.
14 Luftreinigungsvorrichtung nach einer der vorhergehenden Ziffern, bei der stromabwärts des 03-Neutralisierer ein Luftionisierer angeordnet ist.
15 Luftreinigungsvorrichtung nach einer der vorhergehenden Ziffern, bei der stromabwärts des O3-Neutralisierers ein elektrostatischer Luftfilter angeordnet ist.
16 Luftreinigungsvorrichtung nach Ziffer 9, bei der der elektrostatische Luftfilter einander gegenüberstehende elektrisch vorgespannte Elektrodenplatten aufweist, die der Luftstrom passiert.
17 Luftreinigungsvorrichtung nach einer der vorhergehenden Ziffern, deren 03-Erzeuger eine UV-Lichtquelle aufweist.
18 Luftreinigungsvorrichtung nach Ziffer 2 bis 16, deren 03-Erzeuger mindestens ein Koronaelektrodenpaar aufweist, dass an eine Spannungsimpulsversorgung angeschlossen ist.
19 Luftreinigungsvorrichtung nach Ziffer 18, deren Koronaelektrodenpaar Spitzen aufweist, deren Abstand der Impulsspannung angepasst ist.
20 Luftreinigungsvorrichtung nach einer der Ziffern 14 bis 19, deren Luftionisierer Ionisationsdrähte aufweist, die zwischen Hochspannungselektrodenplatten angeordnet sind.
21 Luftreinigungsvorrichtung nach einer der vorhergehenden Ziffern, deren Luftreinigungsvorrichtung einen Luftkühler aufweist.
22 Luftreinigungsvorrichtung nach einer der vorhergehenden Ziffern, deren Luftreinigungsvorrichtung eine Luftheizung aufweist.
23 Luftreinigungsvorrichtung nach einer der vorhergehenden Ziffern, deren Luftreinigungsvorrichtung ein Dreiwegeventil mit zwei Eingängen und einem Ausgang aufweist, wobei an dem Ausgang des Dreiwegeventils der Luftkanal angeordnet ist, und wobei ein erster Eingang mit einer zu reinigenden Raumluft und ein zweiter Eingang über eine Klimaanlage für Luftkühlung und/oder Luftheizung mit einer zu reinigenden Außenluft in Verbindung steht.
24 Ein Luftreinigungsverfahren unter Verwendung einer Luftreinigungsvorrichtung mit einem Gebläse, einem 03-Erzeuger, und einem 03-Neutralisierer weist folgende Verfahrensschritte auf.
   Einschalten des Gebläses, das einen Luftstrom durch einen Luftleitkanal von einer Lufteinlassöffnung zu einer Luftauslassöffnung erzeugt. Leiten des Luftstroms durch einen 03-Erzeuger unter Bilden von Ozonmolekülen, die organische und anorganische Partikel sowie Mikroorganismen zu geruchsneutralen Partikeln oxidieren. Leiten der ozonhaltigen Luft durch einen 03-Neutralisierer unter Reduktion der Ozonrestmoleküle zu geruchsfreien O₂-Sauerstoffmolekühlen.
25 Verfahren nach Ziffer 24, bei dem vor dem Leiten des Luftstroms durch einen 03-Neutralisierer die oxidierten Partikel und oxidierten Mikroorganismen mittels eines Mikrofilters aus dem Luftstrom herausgefiltert werden und das Mikrofilter durch Ozonrestmoleküle entkeimt wird.
26 Verfahren nach Ziffer 24 oder Ziffer 25, bei dem im 03-Neutralisierer Aktivkohle Ozonrestmolekühle reduziert.
27 Verfahren nach Ziffer 24 oder Ziffer 25, bei dem im 03-Neutralisierer Oberflächen von Katalysatormetallen Ozonrestmolekühle zur Reduktion zu geruchsfreien O₂-Sauerstoffmolekühlen veranlassen.
28 Verfahren nach einer der Ziffern 24 bis 27, bei dem im Luftleitkanal der Luftstrom vor dem Austritt aus der Luftauslassöffnung ionisiert wird und submikrometer Partikel und submikrometer Mikroorganismen aus dem Luftstrom entfernt werden.
29 Verfahren nach einer der Ziffern 24 bis 28, bei dem vor dem Austritt des Luftstromes der Ozongehalt des Luftstroms gemessen wird und bei Überschreiten eines vorgegebenen maximalen Ozon-Schwellwertes im Luftrom der 03-Erzeuger abgeschaltet und bei Unterschreiten eines vorgegebenen minimalen Ozon-Schwellwertes den 03-Erzeuger eingeschaltet wird.
30 Verfahren nach einer der Ziffern 24 bis 29, bei dem ein elektrostatischer Luftfilter submikrometer Partikel und submikrometer Mikroorganismen aus dem Luftstrom aufnimmt.
31 Verfahren nach einer der Ziffern 24 bis 30, bei dem der Luftstrom temperiert wird.

### Bezugszeichenliste

- 1: Luftreinigungsvorrichtung (erste Ausführungsform)
- 2: Luftreinigungsvorrichtung (zweite Ausführungsform)
- 3: Luftreinigungsvorrichtung (dritte Ausführungsform)
- 4: Luftreinigungsvorrichtung (vierte Ausführungsform)
- 5: Luftreinigungsvorrichtung (fünfte Ausführungsform)
- 6: Gebläse
- 7: 03-Erzeuger
- 8: 03-Neutralisierer
- 9: Luftleitkanal
- 10: Lufteinlassöffnung
- 11: Luftauslassöffnung
- 12: Luftstrom
- 13: Mikrofilter
- 14: O3-Sensor
- 15: Regler
- 16: Kohlefilter
- 17: Heizeinrichtung
- 18: Luftionisierer
- 19: elektrostatisches Luftfilter
- 20: Elektrodenplatte des Filters (19)
- 21: UV-Lichtquelle
- 22-25: Koronaelektrodenpaar
- 26-33: Spitzen
- 34: Luftkühler
- 35: Luftheizung
- 36: Dreiwegeventil
- 37: erster Eingang
- 38: zweiter Eingang
- 39: Ausgang
- 40: Katalysatormetall
- 41-44: Ionisierungsdraht
- 45-49: Hochspannungsplattenelektrode
- 50: Luftreinigungsvorrichtung (sechste Ausführungsform)

- a: Abstand der Koronaspitzen
- AL: Außenluft
- RL: Raumluft

## Patentansprüche

1. Luftreinigungsvorrichtung mit
- einem.Gebläse (6),
- einem O3-Erzeuger (7),
- einem O3-Neutralisierer (8) und
- einem Luftleitkanal (9) mit
- einer Lufteinlassöffnung (10) und
- einer Luftauslassöffnung (11),
wobei der O3-Erzeuger (7), und der O3-Neutralisierer (8) zwischen Lufteinlassöffnung (10) und Luftauslassöffnung (11) im Luftleitkanal (9) angeordnet und einem Luftstrom (12) des Gebläses (6) ausgesetzt sind,
das Gebläse (6) an der Lufteinlassöffnung (10) oder an der Luftauslassöffnung (11) und der O3-Erzeuger (7) stromaufwärts von dem O3-Neutralisierer (8) im Luftleitkanal (9) angeordnet sind, **dadurch gekennzeichnet, dass** ein Mikrofilter, geeignet zur Entfernung von Partikeln bis zu einer minimalen Größe von 0.3 µm, (13) zwischen dem O3-Erzeuger (7) und dem O3-Neutralisierer (8) angeordnet ist.

2. Luftreinigungsvorrichtung nach Anspruch 1,
**dadurch gekennzeichnet, dass**
die Luftreinigungsvorrichtung einen O3-Sensor (17) aufweist, der stromabwärts des O3-Neutralisierers (8) an der Luftauslassöffnung (11) angeordnet ist, und wobei der O3-Sensor (14) mit einem Regler (15) zusammenwirkt, der bei Überschreiten eines vorgegebenen maximalen Ozon-Schwellwertes im Luftrom (12) an der Luftauslassöffnung (11) den 03-Erzeuger (7) abschaltet und bei Unterschreiten eines vorgegebenen minimalen Ozon-Schwellwertes den 03-Erzeuger (7) einschalter.

3. Luftreinigungsvorrichtung nach einem der vorhergehenden Ansprüche,
**dadurch gekennzeichnet, dass**
der 03-Neutralisierer (8) einen Kohlefilter (16) vorzugsweise aus einem Aktivkohlegranulat aufweist, wobei der Kohlefilter (16) einen offenporigen Körper mit Aktivkohlematerial bildet.

4. Luftreinigungsvorrichtung nach Anspruch 3,
**dadurch gekennzeichnet, dass**
der Kohlefilter (16) mit einer Heizeinrichtung (17) zur Regeneration gekoppelt ist.

5. Luftreinigungsvorrichtung nach einem der Ansprüche 1 bis 2,
**dadurch gekennzeichnet, dass**
der 03-Neutralisierer (6) ein Granulat aufweist, dass mit einem Katalysatormetall (40) beschichtet ist.

6. Luftreinigungsvorrichtung nach einem der Ansprüche 1 bis 2,
**dadurch gekennzeichnet, dass**
der 03-Neutralisierer (8) einen offenporigen Körper aufweist, dessen Poren mit einem Katalysatormetall (40) beschichtet sind.

7. Luftreinigungsvorrichtung nach einem der vorhergehenden Ansprüche,
**dadurch gekennzeichnet, dass**
stromabwärts des O3-Neutralisierer (8) ein Luftionisierer (18) und/oder ein elektrostatischer Luftfilter (19) angeordnet ist.

8. Luftreinigungsvorrichtung nach einem der vorhergehenden Ansprüche,
**dadurch gekennzeichnet, dass**
der 03-Erzeuger (7) eine UV-Lichtquelle (21) oder mindestens ein Koronaelektrodenpaar (22 bis 25) aufweist, dass an eine Spannungsimpulsversorgung angeschlossen ist.

9. Luftreinigungsvorrichtung nach einem der vorhergehenden Ansprüche,
**dadurch gekennzeichnet, dass**
die Luftreinigungsvorrichtung einen Luftionisierer (18) mit Ionisationsdrähten (41 bis 44) aufweist, die zwischen Hochspannungselektrodenplatten (45 bis 49) angeordnet sind.

10. Luftreinigungsvorrichtung nach einem der vorhergehenden Ansprüche,
**dadurch gekennzeichnet, dass**
die Luftreinigungsvorrichtung (5) einen Luftkühler (34) und/oder eine Luftheizung (25) aufweist.

11. Luftreinigungsvorrichtung nach einem der vorhergehenden Ansprüche,
**dadurch gekennzeichnet, dass**
die Luftreinigungsvorrichtung ein Dreiwegeventil (36) mit zwei Eingängen (37, 38) und einem Ausgang (39) aufweist, wobei an dem Ausgang (39) des Dreiwegeventils (36) der Luftkanal (9) angeordnet ist, und wobei ein erster Eingang (37) mit einer zu reinigenden Raumluft (RL) und ein zweiter Eingang (38) über eine Klimaanlage für Luftkühlung und/oder Luftheizung mit einer zu reinigenden Außenluft (AL) in Verbindung steht.

12. Luftreinigungsverfahren unter Verwendung einer Luftreinigungsvorrichtung (1 bis 5) nach den Ansprüchen 1-11 mit einem Gebläse (6), einem O3-Erzeuger (7), und einem 03-Neutralisierer (8),
wobei das Verfahren folgende Verfahrensschritte aufweist:
- Einschalten des Gebläses (6), das einen Luftstrom (12) durch einen Luftleitkanal (9) von einer Lufteinlassöffnung (10) zu einer Luftauslassöffnung (11) erzeugt;
- Leiten des Luftstroms (12) durch einen 03-Erzeuger (7) unter Bilden von Ozonmolekülen, die organische und anorganische Partikel sowie Mikroorganismen zu geruchsneutralen Partikeln oxidieren;
- Leiten der ozonhaltigen Luft durch einen O3-Neutralisierer (8) unter Reduktion der Ozonrestmoleküle zu geruchsfreien O₂-Sauerstoffmolekühlen,
**dadurch gekennzeichnet, dass**
vor dem Leiten des Luftstroms (12) durch einen O3-Neutralisierer (8) die oxidierten Partikel und oxidierten Mikroorganismen mittels eines Mikrofilters (13) aus dem Luftstrom (12) herausgefiltert werden und das Mikrofilter (13) durch Ozonrestmoleküle entkeimt wird.

13. Verfahren nach Anspruch 12,
**dadurch gekennzeichnet, dass**
vor dem Austritt des Luftstromes (12) der Ozongehalt des Luftstroms (12) gemessen wird und bei Überschreiten eines vorgegebenen maximalen Ozon-Schwellwertes im Luftrom (12) der-O3-Erzeuger (7) abgeschaltet und bei Unterschreiten eines vorgegebenen minimalen Ozon-schwellwertes den O3-Erzeuger (7) eingeschaltet wird.

## Claims

1. Air purification device with
- a blower (6),
- an O3 generator (7),
- an O3 neutralizer (8) and
- an air conduction channel (9) with
- an air inlet opening (10) and
- an air outlet opening (11),
wherein the O3 generator (7) and the 03 neutralizer are arranged between the air inlet opening (10) and the air outlet opening (11) in the air conduction channel (9) and are exposed to an air stream (12) of the blower (6), the blower (6) is arranged at the air inlet opening (10) or at the air outlet opening (11) and the O3 generator (7) is arranged upstream of the O3 neutralizer (8) in the air conduction channel (9), **characterized in that**
a micro filter which is suitable for removing particles up to a minimal size of 0.3 micrometers is arranged between the O3 generator (7) and the 03 neutralizer (8).

2. Air purification device according to claim 1,
**characterized in that**
the air purification device comprises an O3-sensor (17), which is arranged downstream of the 03 neutralizer (8) at an air outlet opening (11), and wherein the O3-sensor (14) interacts with a controller (15) which, if a predefined maximum ozone limit is exceeded in the air stream (12) at the air outlet opening (11), switches off the O3 generator (7) and which, if a predefined minimum ozone limit is fallen below, switches on the O3 generator (7).

3. Air purification device according to one of the preceding claims,
**characterized in that**
the 03 neutralizer (8) comprises a carbon filter (16), preferentially of an active carbon granulate, wherein the carbon filter (16) forms an open-pored body with the active carbon material.

4. Air purification device according to claim 3,
**characterized in that**
the carbon filter (16) is coupled with a heating means (17) for regeneration.

5. Air purification device according to one of the claims 1 to 2,
**characterized in that**
the 03 neutralizer (8) comprises a granulate which is coated with a catalyst metal (40).

6. Air purification device according to one of the claims 1 to 2,
**characterized in that**
the 03 neutralizer (8) comprises an open-pored body whose pores are coated with a catalyst metal (40).

7. Air purification device according to one of the preceding claims,
**characterized in that**
downstream of the 03 neutralizer (8) an air ionizer (18) and/or an electrostatic air filter (19) is arranged.

8. Air purification device according to one of the preceding claims,
**characterized in that**
the 03 generator (7) comprises a UV light source (21) or at least one corona electrode pair (22 to 25), which is connected to a voltage pulse supply.

9. Air purification device according to one of the preceding claims,
**characterized in that**
the air purification device comprises an air ionizer (18) with ionizing wires (41 to 44) which are arranged between high voltage electrode plates (45 to 49).

10. Air purification device according to one of the preceding claims,
**characterized in that**
the air purification device (5) comprises an air cooler (34) and/or an air heater (25).

11. Air purification device according to one of the preceding claims,
**characterized in that**
the air purification device comprises a three way valve (36) with two inlets (37, 38) and one outlet (39), wherein the air channel (9) is arranged at the outlet (39) of the three way valve (36) and wherein a first inlet (37) is in connection with a room air (RL) to be cleaned, and a second inlet (38) is in connection with an outside air (AL) to be cleaned via an air-condition for air cooling and/or air heating.

12. Air purification method using an air purification device (1 to 5) according to one of the claims 1 to 11 with a blower (6), an 03 generator (7) and an 03 neutralizer (8) wherein the method comprises the following steps:
- Switching on of the blower (6) which generates an air stream (12) through an air conduction channel (9) from an air inlet opening (10) to an air outlet opening (11);
- Conducting the air stream (12) through an 03 generator (7) under generation of ozone molecules which oxidize organic and inorganic particles as well as micro organisms to odorless particles;
Conducting the ozone containing air through an 03 neutralizer (8) under reduction of the remaining ozone molecules to odorless O2-oxygen molecules,
**characterized in that**
prior to the conducting of the air stream (12) through an 03 neutralizer (8) the oxidized particles and the oxidized micro organisms are filtered out of the air stream (12) via a micro filter (13), and **in that** the micro filter (13) is sterilized through the remaining ozone molecules.

13. Method according to claim 12,
**characterized in that**
prior to the discharge of the air stream (12) the ozone content of the air stream (12) is measured and, if a predefined maximum ozone limit is exceeded in the air stream (12), the ozone generator (7) is switched off and, if the ozone content falls below a predefined minimum, the 03 generator (7) is switched on.

## Revendications

1. Dispositif de purification de l'air avec
- un ventilateur (6),
- un générateur d'ozone (7)
- un neutralisateur d'ozone (8) et
- un conduit à air (9) avec
- une orifice d'admission d'air (10) et
- une orifice de sortie d'air (11),
dans lequel le générateur d'ozone (7) et le neutralisateur d'ozone (8) sont situés entre l'orifice d'admission d'air (10) et l'orifice de sortie d'air (11) dans le conduit à air (9) et ils sont exposés à un flux d'air (12) du ventilateur (6), et le ventilateur (6) est situé à l'orifice d'admission d'air (10) ou à l'orifice de sortie d'air (11), et le générateur d'ozone (8) est situé en amont du neutralisateur d'ozone (8) dans le conduit à air (9), **caractérisé en ce que**
un micro-filtre (13), approprié pour l'extraction de particules jusqu'à une dimension minimale de 0,3 micromètres, est situé entre le générateur d'ozone (7) et le neutralisateur d'ozone (8).

2. Dispositif de purification de l'air selon la revendication 1,
**caractérisé en ce que**
le dispositif de purification de l'air contient un senseur d'ozone (17) qui est situé en aval du neutralisateur d'ozone (8) à l'orifice de sortie d'air (10) et dans lequel le senseur d'ozone (14) interagit avec un régulateur (15), qui arrête le générateur d'ozone (7) quand une limite maximale d'ozone est excédée dans le flux d'air à l'orifice de sortie d'air et qui met en marche le générateur d'ozone quand le teneur en ozone décroît au-dessous une limite minimale d'ozone.

3. Dispositif de purification de l'air selon l'une quelconque des revendications précédentes,
**caractérisé en ce que**
le neutralisateur d'ozone (8) contient un filtre à charbon (16), préférentiellement d'un granulat de charbon actif, dans lequel le filtre à charbon constitue un corps poreux avec un matériel de charbon actif.

4. Dispositif de purification de l'air selon revendication 3,
**caractérisé en ce que**
le filtre à charbon (16) est couplé à un dispositif de chauffage (17) pour la régénération.

5. Dispositif de purification de l'air selon l'une quelconque des revendication 1 à 2,
**caractérisé en ce que**
le neutralisateur d'ozone (8) contient un granulat qui est recouvert d'un métal catalytique (40).

6. Dispositif de purification de l'air selon l'une quelconque des revendications 1 à 2,
**caractérisé en ce que**
le neutralisateur d'ozone (8) contient un corps poreux dont les pores sont recouverts d'un métal catalytique (40).

7. Dispositif de purification de l'air selon l'une quelconque des revendication 1 à 2,
**caractérisé en ce que**
en aval du générateur d'ozone (8) est situé un ionisateur d'air (18) et/ou un filtre à air (19) électrostatique.

8. Dispositif de purification de l'air selon l'une quelconque des revendication précédents,
**caractérisé en ce que**
le générateur d'ozone (7) contient une source de lumière UV (21) ou contient au moins une paire d' électrodes de décharge corona (22 à 25), qui est connectée à une source d'impulsions de tension.

9. Dispositif de purification de l'air selon l'une quelconque des revendication précédents,
**caractérisé en ce que**
le dispositif de purification de l'air contient un ionisateur d'air (18) avec des fils d'ionisation (41 à 44), qui sont connectés entre surfaces d'électrodes de haute tension (45 à 49).

10. Dispositif de purification de l'air selon l'une quelconque des revendication précédents ,
**caractérisé en ce que**
le dispositif de purification de l'air (5) contient un refroidisseur d'air (34) et/ou un chauffage d'air (25) .

11. Dispositif de purification de l'air selon l'une quelconque des revendication précédents,
**caractérisé en ce que**
le dispositif de purification de l'air contient une soupape à trois voies (36) avec deux entrées (37, 38) et une sortie (39), dans lequel le conduit à air (9) est situe à la sortie (39) de la soupape à trois voies (36), et dans lequel une entrée première (37) est en contact avec un air intérieur (RL) à purifier et une entrée seconde (38) est en contact avec un air extérieur (AL) à purifier à travers d'un climatisateur pour refroidissement et/ou chauffage de l'air.

12. Procédé de purification de l'air utilisant un dispositif de purification de l'air (1 à 5) selon l'une quelconque des revendications 1 à 11 avec un ventilateur (6), un générateur d'ozone (7) et un neutralisateur d'ozone (8), le procédé comprenant les étapes suivantes:
- mettre en marche du ventilateur (6), qui produit un flux d'air (12) à travers un conduit à air (9) d'une orifice d'entrée d'air (10) à une orifice de sortie d'air (11);
- conduire le flux d'air (12) au travers d'un générateur d'ozone (7) en formant des molécules d'ozone, qui oxydent à des particules inodores des particules organiques et anorganiques ainsi que des micro-organismes;
- conduire le flux d'air contenant l'ozone au travers d'une neutralisateur d'ozone (8) en réduisant les molécules d'ozone restants à molécules d'oxygène inodores,
**caractérisé en ce que**
avant de conduire le flux d'air (12) au travers d'un neutralisateur d'oxygène (8) les particules oxydées et les micro-organismes oxydées sont filtrés du flux d'air (12) par un micro-filtre (13), et le micro-filtre (13) est stérilisé par les molécules d'ozone restants.

13. Procédé selon revendication 12,
**caractérisé en ce que**
avant de la sortie de flux d'air (12) le contenu en ozone de flux d'air est mesuré et quand une limite maximale d'ozone est excédée dans le flux d'air (12) le générateur d'ozone (7) est arrêté et quand le teneur en ozone décroit au-dessous d'une limite minimale d'ozone le générateur d'ozone est mis en marche.
